# EUROPEAN PATENT APPLICATION

(11) **EP 4 253 521 A1**
(43) Date of publication of application: **04.10.2023**
(21) Application number: 22166393.3
(22) Date of filing: 01.04.2022
(51) Int. Cl.: C12M 1/42, C12M 1/36, G01N 33/50

(54) **LAB-ON-CHIP DESIGNS FOR MEASURING TISSUES**

(71) Applicant: Erasmus University Rotterdam Medical Center, 3015 GE Rotterdam (NL)
(72) Inventor: PIJNAPPEL, Wilhelmus Wenceslaus Matthias, 3015 GE Rotterdam (NL); LULIANO, Alessandro, 3015 GE Rotterdam (NL); DE GREEF, Jessica Christine, 3015 GE Rotterdam (NL)
(74) Representative: V.O.

(57) **Abstract**

A system (100) and method for measuring tissue (T). A chamber (20) is configured to hold a liquid medium (L). Two attachments structures with respective tissue engaging sections (12) are configured to hold the tissue (T) there between inside the chamber (20c) and submerged in the liquid medium (L). At least one of the attachments structures is formed by a cantilever (10). The cantilever (10) comprises a respective bending section (11) formed by an elongate strip with a flat surface (11f) fixated at one end of the cantilever (10) and configured to bend with an, opposite, free end of the cantilever (10) in a direction normal to the flat surface (11f). The respective tissue engaging section (12) is connected at the free end of the cantilever (10) to the respective bending section (11) and configured to hold a respective part of the tissue (T) at said free end.

## Description

### TECHNICAL FIELD AND BACKGROUND

The present disclosure relates to systems and methods for measuring tissue, in particular for lab-on-chip applications such as organ-on-chip and muscle-on-chip.

More than seven hundred human diseases are known that affect skeletal muscle. These can be categorized based on the cause of the disease and include dystrophies, metabolic myopathies, and laminopathies. There is a lack of therapies for skeletal muscle disorders. This is in relevant part due to the current lack of suitable model systems. Muscle biopsies from human patients have been used to culture myoblasts. However, muscle biopsies are invasive, are often contaminated with other cell types, have limited self-renewal capacity, and show reduced capacity to differentiate into myotubes upon passaging. Human myoblasts can be used to generate muscle organoids in vitro that are suitable for functional analysis such as force measurements. Animal models offer an alternative to human biopsies but can never fully recapitulate human disease due to fundamental species-specific differences in muscle homeostasis and regeneration. Furthermore, modeling of human-specific gene mutations is complicated in animal models. An improved option to model human disease is provided by induced pluripotent stem cells (iPSCs). iPSCs have high capacity of self-renewal and provide a virtually unlimited cell source. In principle, the generation of a muscle-on-chip model may alleviate problems of the limited availability of human in vitro models for preclinical research. However, models available to date may lack 3D-muscle structures and cannot measure functional parameters such as force and chemical parameters in situ to monitor pathology and response to treatment in vitro.

As background, WO 2021/251816 describes a method of manufacturing microdevices for lab-on-chip applications. The contents of this previous application are incorporated herein by reference in their entirety. In the known design, the microstructure comprises an elongate chamber with capped micropillars for the generation and/or analysis of muscle tissue. As explained in the previous application, the transition from two to three-dimensional (3D) engineered tissue cultures can change the way biologists perform in vitro functional studies. For example, 3D cell culture systems can be used to mimic the native structure and function of a tissue. Adopting this paradigm is particularly significant for contractile and load-bearing tissues, such as tendons, cardiac and skeletal muscle. For adequate lodging of 3D contractile tissues, flexible pillars are preferred to promote maturation and to provide tendon-like attachment points for contraction. Compared to alternative methods, such as micropatterned surfaces, flexible pillars offer functional advantages in the tracking of their displacement as a consequence of a tissue's contraction. Downscaling the size of engineered tissues is also possible, e.g., by producing pillars in a T-shape. For example, "caps" on top of each pillar can help the retention of smaller tissues under tension.

There is yet a need for further improved designs for lab-on-chip applications, e.g. allowing various tissues to be efficiently and securely provided and/or their properties such as tension and/or contractile forces to be easily and accurately measured.

### SUMMARY

Aspects of the present disclosure relate to systems and methods for measuring tissue. A chamber is configured to hold a liquid medium. One or more cantilevers are configured to hold the tissue, e.g. there between, inside the chamber and submerged in the liquid medium. At least one cantilever comprises a bending section. The bending section is formed by an elongate strip with a flat surface. The bending section is fixated at one end of the cantilever. The other end is configured to bend in a direction normal to the flat surface. A tissue engaging section connected at the free end of the cantilever to the bending section and configured to hold a respective part of the tissue at said free end. Accordingly, the flat surfaces of the respective bending section face the other attachment structure, e.g. second cantilever, for bending towards or away from the other structure depending on a force exerted by contraction or elongation, respectively, of the tissue on the cantilever.

The inventors find that the present disclosed designs of cantilevers having a flat bending section can provide various advantages, e.g. compared to round capped pillars. By providing a flat cantilever strip facing another attachment structure, e.g. second cantilever, a well-defined direction of bending can be provided and the designed bending characteristic thereof can be easily modelled or adapted, e.g. by the thickness and/width of the strip. Accordingly, various forces exerted by the tissue on the cantilever can be more easily and/or accurately measured. By providing each cantilever with a dedicated tissue engaging section, this part can be further tuned to optimize efficiency of growing various tissues and/or keep the tissues securely attached. By making the tissue engaging section relatively wide, e.g. the same width as the strip, preferably wider, a relatively large section is provided to allow tissue engagement. At the same time the strip can remain relatively flat, so the bending accuracy need not be affected.

### BRIEF DESCRIPTION OF DRAWINGS

These and other features, aspects, and advantages of the apparatus, systems and methods of the present disclosure will become better understood from the following description, appended claims, and accompanying drawing wherein:
FIG 1A illustrates a perspective cut-away view of a system for measuring a tissue;
FIG 1B illustrates further aspects of the system in a cross-section view;
FIG 2A illustrates a preferred design of a cantilever;
FIG 2B illustrates further designs of cantilevers;
FIG 3A illustrates various mold shapes to manufacture a chamber;
FIG 3B illustrates a preferred chamber shape.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

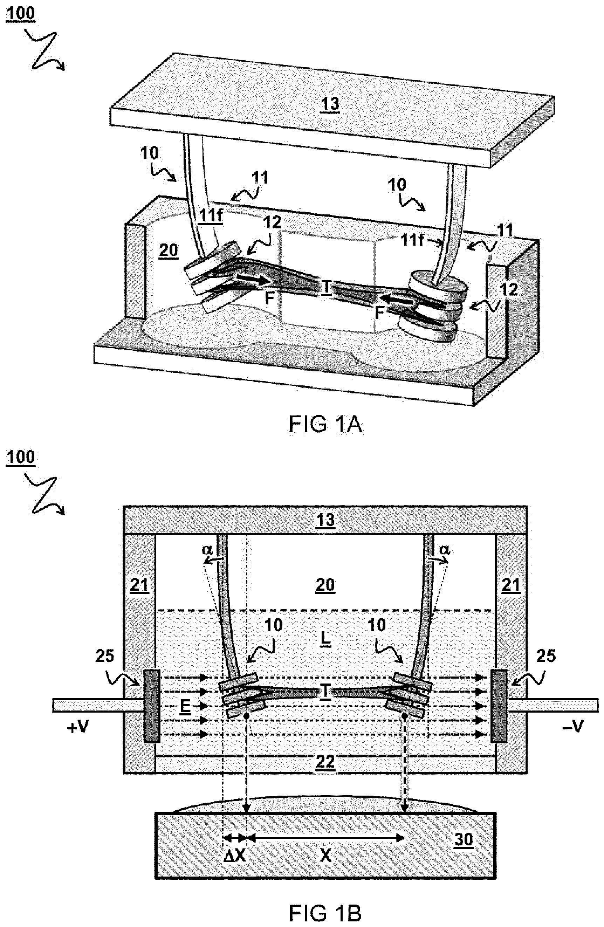

FIG 1A illustrates a perspective cut-away view of a system 100 for measuring a tissue "T". FIG 1B illustrates further aspects of the system 100 in a cross-section view. In some embodiments, a chamber 20 is configured to hold a liquid medium "L". In one embodiment, the liquid medium "L" comprises the tissue "T" and/or precursor cells which may grow and/or combine to form the tissue "T". For example, the chamber 20 is formed by a container having at least a bottom 22 and side walls 21. Also a cover 13 may be provided.

In some embodiments, e.g. as shown, two attachment structures with respective tissue engaging sections 12 are configured to hold the tissue "T" there between inside the chamber 20c and submerged in the liquid medium L. In one embodiment, at least one of the attachments structures is formed by a cantilever 10. In principle, another end of the tissue "T" can be fixed on another attachment structure in the chamber, e.g. a fixated tissue engaging section 12 (not shown). More preferably the two attachment structures are formed by two cantilevers 10, each having the respective tissue engaging section 12 configured to hold the tissue "T" there between.

In some embodiments, at least one cantilever 10 comprises a bending section 11 formed by an elongate strip with a flat surface 11f. In one embodiment, the bending section 11 is fixated at one end of the cantilever 10 and configured to allow an, opposite, free end of the cantilever to bend in a direction normal to the flat surface 11f. In another or further embodiment, a tissue engaging section 12 is connected (at the free end of the cantilever 10) to the bending section 11 and configured to hold a respective part of the tissue "T" at said free end.

In some embodiments, e.g. as shown two cantilevers are provided with respective bending sections 11. In other embodiments (not shown), only one of the cantilevers is provided with a bending section 11, while the other may be provided with a rigid section instead of the bending section. In one embodiment, the thickness of the rigid section in the second cantilever is larger than the bending section in the first cantilever, e.g. by at least a factor two, three, five, ten, or more. In another or further embodiment, the tissue engaging sections 12 of the two cantilevers, or the one cantilever and the second attachment structure, are the same or similar. Advantageously, a configuration with one rigid attachment structure, e.g. rigid cantilever or any other structure, may enhance bending of the remaining one bending cantilever. So, the flat surfaces 11f of the respective bending section 11 may face each other, or face another fixation point for the tissue when a single cantilever is used. Preferably, the bending cantilever may bend towards or away from the other cantilever, or other attachment structure, or they may bend towards each other, depending on a force "F" exerted by contraction or elongation, respectively, of the tissue "T" on the one or more cantilevers 10.

In some embodiments, the one or more cantilevers 10 are fixated at one of their respective ends to a scaffolding structure 13 arranged above the chamber 20. Accordingly, respective free ends of the one or more cantilevers 10 may hang inside the chamber 20 for submerging at least the tissue engaging section 12 in the liquid medium "L". For example, the scaffolding structure 13 comprises a top plate which may also function to close off the chamber. In other or further embodiments (not shown), one or both cantilever 10 can also be held at a fixation point inside the liquid medium "L", e.g. to a bottom plate 22 and/or side wall 25 of the chamber. Also other scaffolding structures can be envisaged e.g. individually holding the respective cantilevers (not shown). In other or further embodiments, at least part of the bending section 11 is arranged above the liquid level. For example, this allows measuring the deflection of the bending section 11 without interference of the liquid medium "L", e.g. by a light beam or other measuring modality.

In some embodiments, the system 100 comprises a measurement device 30 configured to measure a tissue induced change ΔX in distance "X" between respective parts of the attachments structures. For example, the measurement device 30 is configured to measure a distance "X" between the tissue engaging sections 12 which may be formed by the free ends of two cantilevers 10, as shown here, or one cantilever and another attachment point (not shown). Alternatively, or in addition also a distance and/or displacement of any other part of the at least one cantilever can be measured, e.g. displacement of part of the bending section 11. In other or further embodiments, the system 100 comprises a measurement device 30 configured to measure a bending angle "α" of at least one of cantilevers 10. For example, the measurement device 30 is configured to measure a relative angle "α" of a part of the bending section 11. In some embodiments, the measured a distance "X", displacement "ΔX", angle "α", or any other measured characteristic of the tissue induced bending, is used to determine a force "F" applied by the tissue "T" on the one or more cantilevers 10 (e.g. contractile force applied by the tissue on the cantilevers) and/or vice versa (e.g. stretching force applied by the one or more cantilevers on the tissue).

In some embodiments, the chamber 20 has a transparent bottom and/or side wall. In one embodiment, e.g. as shown, the chamber 20 has a transparent bottom plate 22 to allow viewing the cantilevers 10 by a measurement device 30 such as a camera or other imaging and/or projection device. Also other or further measuring devices and/or modalities can be envisaged to measure the bending of the cantilevers.

In other or further embodiments, (not shown), the cantilever 10 comprises a piezoelectric material on one or both surfaces of the bending section 11. In one embodiment, an electric sensor is configured to measure an electrical signal from the piezoelectric material to determine the bending of the cantilever, e.g. caused by a force "F" exerted by the tissue on the cantilever(s). For example, an electric signal such as current and/or voltage is measured resulting from compression and/or extension of the piezoelectric material on either side of the bending section 11. In another or further embodiment, an electric source is configured to generate and/or apply an electric signal to the piezoelectric material to cause a force and/or bending of the cantilever. For example, this can be used to stimulate the tissue by forcing elongation and/or contraction.

In other or further embodiments (not shown), the system is configured to illuminate part of the cantilever 10, e.g. the flat surface 11f, to measure a position, displacement and/or angle of said part. In one embodiment, the system is configured to measure a reflected light beam to determine an angular deflection and/or phase of the reflected light beam as a result of a bending of the cantilevers 10. In principle, the bending can also be determined by measuring deflection of a light beam transmitted through a transparent part of the cantilever 10. In other or further embodiments, the system comprises a light source to generate the light beam. In another or further embodiment (not shown), the system comprises one or more sensors to measure a reflected light beam. In some embodiments, an amount of light measured by the one or more sensors is used to determine the bending of the cantilevers. In one embodiment, the light source comprises a laser configured to direct a light beam onto the cantilever 10, e.g. a reflecting part of the bending section 11. In another or further embodiment, the system comprises beam shaping and/or light guiding means to direct the light beam onto the cantilever 10 and/or guide light back to a sensor. In one embodiment, at least one optical fiber is configured to measure deflection of a respective cantilever. For example, one or more optical fibers may have an entry and/or exit port inside the chamber. In another or further embodiment, bending is measured using a reflection and/or transmission or reflection of the light from the cantilever. For example, an origin fiber is oriented perpendicular to a reflecting surface, e.g. formed by part the bending section in rest position. In this case, the more the cantilever is bent, the less light may be reflected back in to the origin fiber. Alternatively, or additionally, more light may be received in the origin fiber if it is oriented at an angle with respect to the surface normal. Alternatively, or additionally, more or less light may be reflected into a second fiber configured to receive (reflected and/or transmitted) light from the bending cantilever. Alternatively, or additionally, a phase of light reflected back into the first and/or second fiber can be measured, e.g. interferometrically, to determine the bending. Alternatively, or in addition to reflecting light off the cantilever 10, also other light-based techniques can be used. For example, a Fiber Bragg Grating (FBG) can be embedded in the cantilever, or attached to the cantilever, and an optical interrogator configured to measure tension in said FBG to derive bending and/or tension on the cantilever.

In some embodiments, (not shown), the system comprises a controller and/or processor configured to calculate the force "F" based on at least one of the distance "X", the displacement "ΔX", the bending angle "α", an electrical and/or light signal. Any of the measured quantities, such as electricity, light, distance "X", angle "α", et cetera, can be directly converted to an associated force "F", e.g. using a predetermined relation between respective forces and measurement and/or a model of this relation can be used.

In some embodiments, the system comprises a set of electrodes arranged to generate and electric field "E" inside the chamber 20. In one embodiment, the electric field "E" is configured to stimulating the tissue "T", e.g. stimulating contraction of muscle tissue. Also other or further types of actuation can be used, e.g. (piezoelectric) actuator(s) and/or magnetic actuators configured to apply a known and/or calibrated force to the free ends of the cantilevers to move them apart or together with tissue T" there between. In one embodiment, at least a part of the electrodes disposed in the chamber comprises, is covered by, or essentially consists of carbon. This may prevent oxidation and toxic products in the medium, and wear and tear of the electrodes.

In some embodiments, e.g. as shown, the tissue engaging sections 12 (e.g. at the free ends of the cantilevers 10) are disposed between the electrode 25. For example, the tissue engaging sections are arranged on (direct) line between the electrodes. Preferably, the electrodes are configured to generate an electric field along a length of the tissue, i.e. between the cantilevers. Typically, a distance between the electrodes is in a range between 0.5 and 5 cm, preferably between one and three centimeter. It will be understood that the strength of the electric field "E" may depend on the voltage difference (±V) divided by the distance between the electrodes. For example, an applied voltage may be in a range between one and one hundred volt per centimeter, preferably in a range between two and twenty volt per centimeter, most preferably between three and ten volt per centimeter. For example, 4.5 V is applied over a distance of 1 cm.

Also combinations of the described techniques are possible, e.g. a selection of two or more modalities from a camera based measurement, light reflection and/or transmission based measurement, a piezoelectric based measurement and/or actuation of tissue, actuation of tissue using an electric field, other types of actuation and/or measurement, et cetera. In some embodiments, a controller and/or processor (not shown) is configured to determine (measure and/or cause) a change ΔX in distance "X" and/or bending angle "α" and/or amount of light and/or electrical signal as function of one or more voltages applied between the electrodes 25 and/or electrical signals applied to piezoelectric materials on the cantilevers, and optionally calculate the force "F" on the basis thereof. For example, a set of different voltages is applied and a set of corresponding measurements of the resulting bending of at least one cantilever is recorded. In one embodiment, the controller comprises or access a non-transitory computer-readable medium storing instructions that, when executed by one or more processors, cause the system to perform operational acts according to one or more or the methods described herein

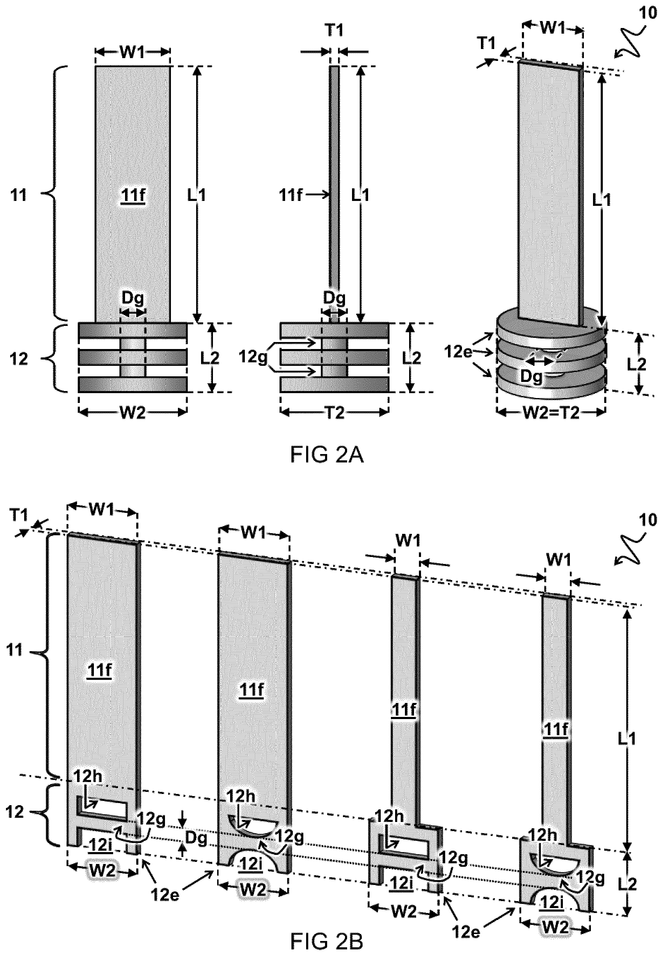

FIG 2A illustrates different views of a preferred design for the cantilever 10. FIG 2B illustrates further designs of cantilevers 10. As illustrated for each of the designs, the elongate flat surface 11f of the bending section 11 has a respective length L1, width W1, and thickness T1 (defined in perpendicular directions with respect to each other). In a preferred embodiment, the width W1 is larger than the thickness T1, e.g. by at least a factor two, three, five, ten, or more. For example, the width W1 of the bending section 11 is between 0.2 and 5 mm, more preferably between 0.5 and 3 mm, most preferably less than two millimeter, e.g. one millimeter, or less. Preferably, the thickness T1 of the bending section 11 is between 0.01 and 1 mm, more preferably between 0.05 - 0.05 mm, e.g. 0.1 mm, or less. The thinner and/or less wide the bending section, the more sensitive the cantilever may react to a force "F" of the tissue "T". Keeping the cantilever wider than the thickness, may improve predictable bending behavior and/or manufacturability. In other or further embodiments, e.g. as illustrated for each of the designs, the length L1 of the bending section 11 is larger than the width W1 of the bending section 11, e.g. by at least a factor two, three, five, or more. For example, the length L1 of the bending section 11 is more than five millimeter, preferably more than one centimeter, e.g. up to two or three centimeter, or more.

In some embodiments, e.g. as illustrated in FIG 2A, and for the third and fourth designs in FIG 2B, the tissue engaging section 12 has a relatively large width W2 compared to the width W1 of the bending section 11, e.g. larger by at least ten percent, preferably larger by at least fifty percent, more preferably at least a factor two, three, or more. For example, the width W2 of the tissue engaging section 12 is between one and ten millimeter, more preferably between 1.5 and 5 mm, most preferably more than two millimeter, e.g. three millimeter. By having a relatively wide tissue engaging section 12, there may be more room for engaging tissue "T", while keeping the bending section 11 relatively flexible.

In a preferred embodiment, e.g. as illustrated for the design in FIG 2A, the tissue engaging section 12 has three dimensional shape. Accordingly, the tissue engaging section 12 can have a relatively large thickness T2 compared to a thickness T1 of the bending section 11, e.g. larger by at least ten percent, preferably larger by at least fifty percent, more preferably at least a factor two, three, five, ten, or more. In another or further preferred embodiment, the thickness T2 of the tissue engaging section 12 is the same or similar as its width W2, e.g. the same within ±50%. Most preferably, the tissue engaging section comprises a cylindrical shape, e.g. wherein the thickness T2 and width W2 are defined by a diameter of the cylinder. Alternative, to the shown cylindrical shape also other shapes can be envisaged for a tissue engaging section 12 having a three dimensional shape with a relatively large thickness T2, e.g. a locally thickened block shape. Advantageously, a three dimensional shaped tissue engaging section 12 may provide an improved physiological model for engaging tissue. In one embodiment, e.g. as shown, the tissue engaging section 12 comprises a set of two, three, or more cylindrical shapes forming respective protruding extremities 12e with one, two, or more circumferential slits there between configured to engage the tissue. As will be appreciated, the free end of cantilever 10 may appear similar to a honey spoon and similarly provide a relatively large surface area for tissue to grow and/or engage.

In other embodiments, e.g. as illustrated for each of the designs in FIG 2B, the tissue engaging section can also have a flat shape, e.g. with the same thickness T1 as the bending section 11. This may provide advantages of manufacturability. Preferably, the flat formed tissue engaging section is still wider than the bending section, e.g. according to the relative widths W2/W1 indicated earlier and shown for the two designs on the right hand side of FIG 2B. Most preferably, the flat formed tissue engaging section 12 has at least one hole 12h through its flat surface to facilitate tissue connection, e.g. through the hole. In one embodiment, e.g. as illustrated in the first and third designs of FIG 2B, the hole 12h has rectangular shape. More preferably, e.g. as illustrated in the second and fourth design of FIG 2B, the hole 12h has a rounded shape, e.g. concave towards the free end of the cantilever 10.

In a preferred embodiment, e.g. as illustrated for each of the designs in FIGs 2A and 2B, the tissue engaging section 12 comprises at least one grip structure 12g, formed between protruding extremities 12e and/or a hole 12h through the cantilever 10. For example, the grip structure 12g may facilitate an end of the tissue "T" to wrap around and/or facilitate holding the tissue "T" between the protruding extremities 12e and/or through the hole 12h. In some embodiments, e.g. as illustrated for the design of FIG 2A, the grip structure 12g is formed by the relatively narrow parts connecting the protruding cylindrical shapes, i.e. extremities 12e. In other embodiments, e.g. as illustrated for the designs of FIG 2B, the grip structure 12g is formed in a direction of the width W2 between the hole 12h and the free end of the cantilever 10. Most preferably, e.g. as illustrated for each of the designs in FIGs 2A and 2B, the grip structure 12g has a relatively small diameter Dg and/or circumference (not indicated), so the tissue "T" can more easily wrap around this grip structure 12g, while it is prevented from slipping off by the protruding extremities and/or by looping through a hole 12h. For example, the grip structure 12g has maximum grip diameter Dg that is the same or similar as the thickness T1 of the bending section 11, e.g. within a factor ten, preferably within a factor five or within a factor three.

In some embodiments, e.g. as illustrated for the designs in FIG 2B, the free end of the cantilever 10 has a concave (preferably rounded) shape formed along the width W2 of the tissue engaging section 12 between two protruding extremities 12e at respective sides of said width W2 and cutting into the free end of the cantilever 10 towards the hole 12h to form an elongate grip section 12g.

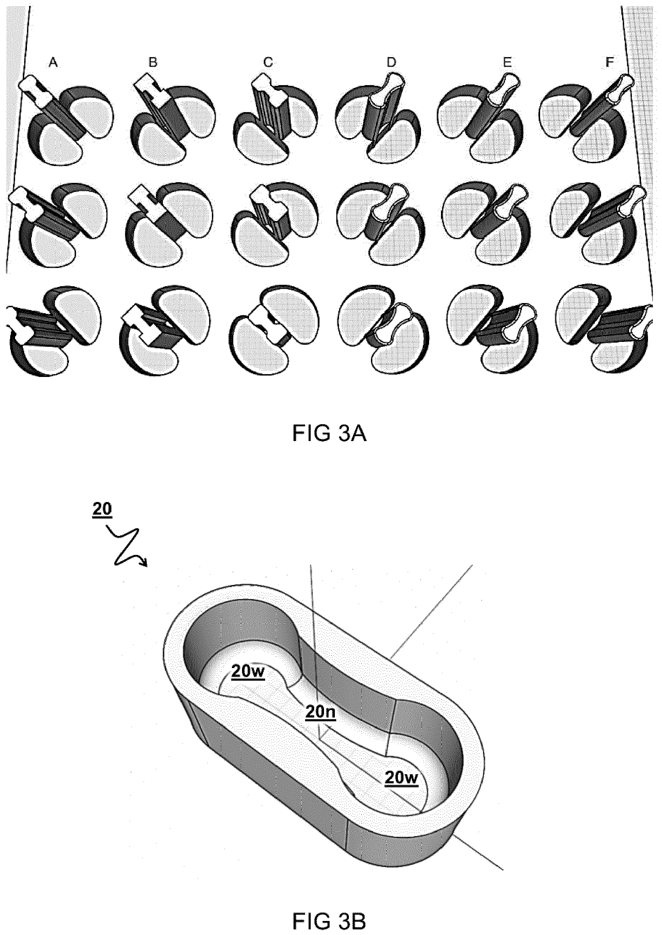

FIG 3A illustrates various mold shapes to manufacture a chamber 20 (negative of the mold shape). FIG 3B illustrates a preferred chamber shape. In some embodiments, e.g. as illustrated, chamber has two relatively wide ends 20w (where the cantilevers are disposed) and a relatively narrow midsection 20n for holding the tissue there between. For example, the chamber has a sort of bone shape. Advantageously, this may allow sufficient room for a relatively wide and/or three dimensional the tissue engaging section while the volume of liquid medium can be kept relatively low. Accordingly, less cellular material may be needed. Most preferably, the chamber is provided with rounded walls without corners, e.g. to optimally accommodate the cantilever design of FIG 2A and/or prevent tissue sticking in the corners.

Aspects, as described herein, can also be embodied in corresponding methods for measuring tissue. In one embodiment, the system 100 as described is provided with a tissue "T" between respective tissue engaging sections 12 in the chamber 20 filled with liquid medium L, e.g. free ends of one or more cantilevers 10 in the chamber 20 filled with liquid medium "L". In another or further embodiment, a bending of one or more cantilevers is measured. In another or further embodiment, a force exerted between the at least one cantilever and the tissue, or vice versa, is calculated based on the measured deflection. For example, the tissue "T" comprise contractile tissue such as muscle, e.g. skeletal muscle, smooth muscle, cardiac muscle. Also other tissues can be used, e.g. tendon. Alternatively, or in addition to measuring a force, a force can also be applied to the tissue, e.g. applying a stretching force by the cantilevers via piezo, magnetic et cetera.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. A system (100) for measuring tissue (T), the system comprising
a chamber (20) configured to hold a liquid medium (L); and
two attachments structures with respective tissue engaging sections (12) configured to hold the tissue (T) there between inside the chamber (20c) and submerged in the liquid medium (L);
wherein at least one of the attachments structures is formed by a cantilever (10) comprising
a respective bending section (11) formed by an elongate strip with a flat surface (11f) fixated at one end of the cantilever (10) and configured to bend with an, opposite, free end of the cantilever (10) in a direction normal to the flat surface (11f), and
the respective tissue engaging section (12) connected at the free end of the cantilever (10) to the respective bending section (11) and configured to hold a respective part of the tissue (T) at said free end.

2. The system according to the preceding claim, wherein the two attachment structures are formed by two cantilevers (10), each having the respective tissue engaging section (12) configured to hold the tissue (T) there between, wherein at least the first cantilever has the respective bending section (11) with the flat surface (11f) facing the second cantilever for bending towards or away from the second cantilever depending on a force (F) exerted by contraction or elongation, respectively, of the tissue (T) on the cantilevers (10).

3. The system according to any of the preceding claims, wherein the elongate strip with flat surface (11f) of the bending section (11) has a respective length (L1), width (W1), and thickness (T1), wherein the width (W1) is larger than the thickness (T1) by at least a factor two, and the length (L1) of the bending section (11) is larger than the width (W1) of the bending section (11) by at least a factor two.

4. The system according to the preceding claim, wherein a width (W2) of the tissue engaging section (12) is larger than a width (W1) of the bending section (11) by at least fifty percent.

5. The system according to any of the preceding claims, wherein the tissue engaging section (12) has three dimensional shape with a thickness (T2) that is larger a thickness (T1) of the bending section (11) by at least a factor two.

6. The system according to any of the preceding claims, wherein the tissue engaging section (12) comprises a set of cylindrical shapes forming respective protruding extremities (12e) with one, two, or more circumferential slits forming a respective grip structure (12g) there between configured to engage the tissue (T).

7. The system according to any of claims 1-5, wherein the tissue engaging section (12) is flat and has the same thickness (T1) as the bending section (11), wherein the flat formed tissue engaging section has at least one hole (12h) through its flat surface to facilitate tissue connection through the hole (12h), wherein the free end of the cantilever (10) has a concave, preferably rounded, shape formed along the width (W2) of the tissue engaging section (12) between two protruding extremities (12e) at respective sides of said width (W2) and cutting into the free end of the cantilever (10) towards the hole (12h) to form an elongate grip section (12g).

8. The system according to any of claims 6 or 7, wherein the grip structure (12g) is configured to wrap an end of the tissue (T) around and hold the tissue (T) between the protruding extremities (12e) and/or through the hole (12h), wherein the grip structure (12g) has maximum grip diameter (Dg) that is the same or similar as the thickness (T1) of the bending section (11) within a factor ten.

9. The system according to any of the preceding claims, comprising
a measurement device (30) configured to measure a tissue induced displacement (ΔX) and/or bending (a) of at least one cantilever (10), to determine a force (F) between the tissue (T) and the at least one cantilever (10); and
a processor configured to calculate the force (F) based on the measurement.

10. The system according to the preceding claim, wherein the chamber (20) has a transparent wall, wherein the measurement device (30) comprise a camera configure to image at least part of the at least one cantilever (10).

11. The system according to any of the preceding claims, wherein at least one cantilever (10) comprises a piezoelectric layer and the system comprises an electric transceiver configured to measure and/or induce a bending of the cantilever (10) by measuring and/or supplying an electric signal to the piezoelectric layer.

12. The system according to any of the preceding claims, wherein the system comprises a set of electrodes (25) arranged to generate an electric field (E) inside the chamber (20) for stimulating the tissue (T), wherein at least a part of the electrodes disposed in the chamber is covered by, or consists of carbon, wherein the tissue engaging sections (12) are disposed between the electrodes (25), and the electrodes are configured to generate an electric field along a length of the tissue (T) between the tissue engaging sections (12), the system further comprising a controller configured to apply one more voltages applied between the electrodes (25), and at least one sensor configured to determine the bending of the at least one cantilever as function of the one or more voltages applied between the electrodes (25).

13. The system according to any of the preceding claims, wherein two cantilevers (10) are fixated at one of their respective ends to a scaffolding structure (13) arranged above the chamber (20) with respective free ends of the cantilevers (10) hanging inside the chamber (20) for submerging at least the tissue engaging section (12) in the liquid medium (L), wherein the chamber (20) has bone shape with two relatively wide rounded ends (20w), where the cantilevers are hanging, and a relatively narrow midsection (20n) for holding the tissue (T) there between.

14. A method for measuring tissue (T), the method comprising
providing the system (100) according to any of the preceding claims with a tissue (T) between respective tissue engaging sections (12) in the chamber (20) filled with liquid medium (L); and
measuring and/or actuating a bending of at least one cantilever (10) holding a respective part of the tissue (T) to measure and/or cause a force exerted between the cantilever (10) and the tissue (T).

15. The method according to the preceding claim, wherein the tissue (T) comprises a contractile tissue such as muscle.
